(19) Europäisches Patentamt
European Patent Office
Office européen des brevets

(11) **EP 4 525 796 B1**

(12) **EUROPÄISCHE PATENTSCHRIFT**

(45) Veröffentlichungstag und Bekanntmachung des Hinweises auf die Patenterteilung:
**29.04.2026 Patentblatt 2026/18**

(21) Anmeldenummer: **23716207.8**

(22) Anmeldetag: **28.03.2023**

(51) Internationale Patentklassifikation (IPC):
*A61F 13/01* (2024.01)  *D04H 1/407* (2012.01)
*D04H 1/46* (2012.01)  *D04H 1/541* (2012.01)
*D04H 1/4391* (2012.01)  *A61L 15/60* (2006.01)
*A61L 15/42* (2006.01)  *A61L 15/28* (2006.01)

(52) Gemeinsame Patentklassifikation (CPC):
**D04H 1/407; A61F 13/01012; A61F 13/01017;
A61L 15/28; A61L 15/42; A61L 15/60;
D04H 1/43918; D04H 1/46; D04H 1/5414**

(86) Internationale Anmeldenummer:
**PCT/EP2023/057955**

(87) Internationale Veröffentlichungsnummer:
**WO 2023/222291 (23.11.2023 Gazette 2023/47)**

(54) **WUNDAUFLAGE**

WOUND DRESSING

PANSEMENT POUR PLAIES

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorität: **19.05.2022 DE 102022112586**

(43) Veröffentlichungstag der Anmeldung:
**26.03.2025 Patentblatt 2025/13**

(73) Patentinhaber: **Carl Freudenberg KG
69469 Weinheim (DE)**

(72) Erfinder:
• **PEHR, Marc
69514 Laudenbach (DE)**
• **SCHLESSELMANN, Bernd
69469 Weinheim (DE)**
• **DUNCKER-RAKOW, Samuel
64625 Bensheim (DE)**

(56) Entgegenhaltungen:
DE-A1- 102007 049 429    DE-A1- 102017 001 928
JP-A- H0 657 610    US-A- 5 454 142

**Beschreibung**

Die Erfindung betrifft eine Wundauflage, umfassend einen Nadelvliesstoff, der superabsorbierende Fasern enthält, sowie ein Verfahren zu seiner Herstellung. Die Erfindung betrifft ferner die Verwendung eines Nadelvliesstoffs, der superabsorbierende Fasern enthält, zur Herstellung einer Wundauflage.

Hochabsorbierende Vliesstoffe, die Superabsorberfasern enthalten, sind allgemein bekannt. Sie werden häufig als Komponenten in Wundauflagen eingesetzt, da sie über ein hohes Aufnahmevermögen und Rückhaltevermögen gegenüber Wundflüssigkeit verfügen. Darüber hinaus sind sie aufgrund ihrer textilen Eigenschaften anpassungsfähig und können damit zu einem hohen Tragekomfort der Wundauflage beitragen.

Nachteilig an solchen Superabsorberfaser enthaltenden Vliesstoffen ist allerdings, dass sie nicht reversibel expandierbar sind, das heißt, dass sie nicht oder nur kaum elastisch auf Zug reagieren. Hierdurch können z.B. durch Bewegungen des Patienten verursachte Kräfte zu einem Verlust der Haftung oder einem Verrutschen der Wundauflage führen. Dies gilt insbesondere, wenn die Wundauflage auf oder in der Umgebung von einem Gelenk eingesetzt wird. Falls die Komponenten der Wundauflage über unterschiedliche Elastizitäten verfügen, kann es zudem zu einer Delaminierung der Komponenten kommen.

Ein weiterer Nachteil von Superabsorberfaser enthaltenden Vliesstoffen ist, dass sie bei Kontakt mit Luftfeuchtigkeit während der Lagerung und bei der Aufnahme von Flüssigkeit während der Anwendung schrumpfen. Wird ein solcher Vliesstoff als Komponente in einer Wundauflage eingesetzt, die zusätzlich andere, nicht-schrumpfende, Lagen enthält, dann kann der Schrumpf des Vliesstoffs zu Faltenbildung führen, oder zu einer Deformation der gesamten Wundauflage.

Darüber hinaus verlieren Superabsorberfasern, insbesondere wenn sie aus Polyacrylat oder Polymethacrylat bestehen, bei Aufnahme von wässrigen Flüssigkeiten in der Regel ihre Integrität, und das dabei gebildete Hydrogel ist nicht stabil gegen mechanische Einflüsse in den Vliesstoff eingebunden. Bereits durch geringe mechanische Beanspruchung können sich Gelpartikel aus dem Vliesstoff lösen.

Es wurden bereits verschiedene Ansätze vorgeschlagen, um die absorbierenden Komponenten einer Wundauflage mit elastischen Eigenschaften zu versehen. Diese erfordern jedoch häufig den Einsatz elastomerer Werkstoffe oder aufwändiger Herstellungsverfahren. Ferner bewirken manche dieser Ansätze nur Elastizität in eine Dimension. So beschreibt die WO2010/035017A1 eine Fadenverstärkung mit elastischem Garn. Die damit gewonnene Elastizität wirkt jedoch nur in Längsrichtung. Die EP3666295A1 beschreibt die Anbindung eines absorbierenden Vliesstoffs an ein Flächengebilde aus elastomerem Material. Dies ist aufwändig, und die so erzielte Elastizität wirkt nicht homogen über die Dicke des resultierenden Materials.

EP3285705B1 beschreibt eine Wundauflage mit einer Faserlage, welche mit Gruppen von Einschnitten versehen wird und dadurch eine erhöhte Flexibilität aufweist. Die auf diese Weise verformbar gemachte Lage zeigt jedoch nur geringe elastische Rückerholung.

DE 10 2007 049429 A1 beschreibt eine flächige Wundauflage die einen Vliesstoff umfasst. Dieser Vliesstoff enthält superabsorbierende Fasern sowie stützende Fasern, wobei die stutzenden Fasern auch im feuchtem Zustand die Integrität der Wundauflage gewährleisten.

Ebenfalls bekannt sind elastische Vliesstoffe, die aus latent selbstkräuselnden Fasern hergestellt sind. So beschreibt US5454142A ein Verfahren zur Herstellung eines Vliesstoffs aus Fasern mit einer mechanischen Kräuselung und einer latenten Kräuselung (Selbstkräuselung). Dabei wird der Vliesstoff zum Ausbilden der latenten Kräuselung erhitzt. Die auf diese Weise hergestellten Vliesstoffe zeigen sehr gute elastomere und schaumartige Kompressibilitäts- und Elastizitätseigenschaften. Die Fasern sind aus thermoplastischen Materialien und insbesondere aus Polyester und weisen keine oder nur geringe absorbierende Eigenschaften auf. Deshalb sind sie für saugfähige Komponenten in Wundauflagen nicht geeignet.

Der Erfindung liegt die Aufgabe zugrunde eine Wundauflage bereit zu stellen, die eine hohe Elastizität mit einer guten Absorptionsfähigkeit kombiniert, die gleichzeitig nach Absorption von Flüssigkeit stabil gegen mechanische Einflüsse ist, keinen oder einen nur geringen Schrumpf bei Kontakt mit Flüssigkeit oder mit Luftfeuchtigkeit während der Lagerung aufweist und gel-blockierende Eigenschaften zeigt. Weitere Aufgaben liegen darin, ein Verfahren zur Herstellung der Wundauflage bereitzustellen sowie die Verwendung eines Vliesstoffs zu ihrer Herstellung.

Eine Aufgabe der Erfindung wird gelöst durch eine Wundauflage umfassend einen Nadelvliesstoff enthaltend:

- 30 Gew.% bis 80 Gew.%, bevorzugt 40 Gew.% bis 75 Gew.%, noch bevorzugter 45 Gew.% bis 70 Gew.%, insbesondere 55 Gew.% bis 65 Gew.%, bezogen auf das Gesamtgewicht des Nadelvliesstoffs, superabsorbierende Fasern;

- 70 Gew.% bis 20 Gew.%, bevorzugt 60 Gew.% bis 25 Gew.%, noch bevorzugter 55 Gew.% bis 30 Gew.%, insbesondere 45 Gew.% bis 35 Gew.% bezogen auf das Gesamtgewicht des Nadelvliesstoffs, selbstgekräuselte Fasern.

Die erfindungsgemäße Wundauflage zeichnet sich durch eine hohe Elastizität kombiniert mit einer guten Absorptionsfähigkeit aus. Gleichzeitig ist sie stabil gegen mechanische Einflüsse und weist nach Absorption von Flüssigkeit oder bei Kontakt mit Luftfeuchtigkeit während der Lagerung keinen oder einen nur geringen Schrumpf sowie gel-blockierende Eigenschaften auf.

Ohne sich auf einen Mechanismus festzulegen, wird vermutet, dass diese vorteilhaften Eigenschaften darauf zurückzuführen sind, dass die selbstgekräuselten Fasern dazu in der Lage sind die superabsorbierenden Fasern besonders gut in ihre Gerüststruktur einzubinden und dadurch zu stabilisieren. Hierdurch kann das bei der Absorption von wässrigen Flüssigkeiten durch die superabsorbierenden Fasern gebildete Gel stabil gegen mechanische Einflüsse in den Nadelvliesstoff eingebunden werden. Dabei war insbesondere überraschend, dass bereits mit einem Minderheitsanteil an selbstgekräuselten Fasern eine gute Einbindung des Gels in Kombination mit elastischer Rückerholung erhalten werden kann. Darüber hinaus wurde gefunden, dass die erfindungsgemäße Wundauflage gel-blockierende Eigenschaften zeigt. Dies ist vorteilhaft bei Wundauflagen, weil dabei der Flüssigkeitstransport in vertikaler Richtung über den Wundrand hinaus behindert wird und somit die unverletzte Haut weniger Flüssigkeit ausgesetzt wird, was wiederum das Auftreten von Mazeration verhindert. Es wird vermutet, dass dieser Effekt dadurch zustande kommt, dass die selbstkräuselnden Fasern zu einer Kompaktierung des Nadelvliesstoffs führen, wodurch weniger freies Volumen zum Flüssigkeitstransport bereitsteht.

Unter selbstgekräuselten Fasern sind Fasern zu verstehen, die aus selbstkräuselnden Fasern (auch latent kräuselnde Fasern genannt) hergestellt werden. Ihre Kräuselung kann durch thermisches Auslösen der latenten Kräuselungsfähigkeit der als Ausgangsmaterial verwendeten selbstkräuselnden Fasern erhalten werden. Vorzugsweise ist der Nadelvliesstoff mithin thermisch behandelt. Vorteilhaft an der Verwendung von selbstgekräuselten Fasern ist, dass sie dem Nadelvliesstoff eine hohe Elastizität verleihen.

Bevorzugt weisen die selbstgekräuselten Fasern Fasermaterialien auf, die ausgewählt sind aus der Gruppe der Polyamide, beispielsweise Polyamid 6, Polyamid 6,6 oder Polyamid 11, der Polyester, beispielsweise Polyethylenterephthalat (PET) oder Polybutylenterephthalat (PBT), Polybutylensuccinat (PBS), und der Polyolefine, beispielsweise Polypropylen (PP) oder Polyethylen (PE), oder aus deren Gemischen oder Copolymeren.

Bevorzugte selbstgekräuselte Fasern sind hergestellt aus selbstkräuselnden Fasern, die mindestens zwei Komponenten aufweisen, wobei eine Komponente ein anderes Schrumpfverhalten unter thermischer Behandlung aufweist als die andere Komponente und wobei die Anordnung der Komponenten eine dreidimensionale Verformung der Faser induziert, zum Beispiel in Form einer Spirale oder Helix, oder in einer unregelmäßigen von der Geraden abweichenden Struktur.

Ebenfalls bevorzugte selbstgekräuselte Fasern sind hergestellt aus selbstkräuselnden Fasern, bei denen ein unterschiedliches Schrumpfverhalten unter thermischer Behandlung mindestens einer Faserkomponente gegenüber mindestens einer anderen Faserkomponente vorliegt. Vorteilhafterweise wird dies durch Verwendung von unterschiedlichen Polymeren, die sich in ihrer Morphologie unterscheiden, erreicht. Beispielsweise kann ein Homopolymer als eine Komponente und ein Co-Polymer als eine andere Komponente verwendet werden. Alternativ kann ein Morphologieunterschied erzeugt werden durch unterschiedliche Molekulargewichte, Stereochemie, Verzweigungsgrad oder Vernetzung. Typischerweise weisen die selbstkräuselnden Fasern eine erste Faserkomponente und eine zweite Faserkomponente auf.

Bevorzugt weisen die Faserkomponenten der selbstkräuselnden Fasern, insbesondere die erste Komponente und die zweite Faserkomponente, Fasermaterialien auf, die unabhängig voneinander ausgewählt sind aus der Gruppe der Polyamide, beispielsweise Polyamid 6, Polyamid 6,6 oder Polyamid 11, der Polyester, beispielsweise Polyethylenterephthalat (PET) oder Polybutylenterephthalat (PBT), Polybutylensuccinat (PBS), und der Polyolefine, beispielsweise Polypropylen (PP) oder Polyethylen (PE), oder aus deren Gemischen oder Copolymeren.

Die selbstgekräuselten Fasern und/oder die als Ausgangsmaterial verwendeten selbstkräuselnden Fasern können als Bikomponenten-Fasern vorliegen, in denen die Faserkomponenten beispielsweise in Seite-an-Seite-Anordnung mit insgesamt rundem Querschnitt, oder in exzentrischer Kern-Mantel-Anordnung mit insgesamt rundem Querschnitt vorliegen. Ein Kräuselungspotential kann jedoch auch mit anderen im Stand der Technik bekannten Querschnitten und Bikomponenten-Formen erzielt werden.

Wie oben dargelegt, sind die erfindungsgemäß verwendeten selbstgekräuselten Fasern aus selbstkräuselnden Fasern hergestellt, deren Kräuselung durch thermisches Auslösen ihrer latenten Kräuselungsfähigkeit erhalten werden kann. Dies hat den Vorteil, dass es möglich ist die Kräuselung erst nach Vliesstoffherstellung durchzuführen, was wiederum vorteilhaft ist, weil dadurch die Ausbildung elastischer Rückerholung ermöglicht wird und gleichzeitig eine gute Einbindung der superabsorbierenden Fasern erfolgt.

Unabhängig von ihrer Selbstkräuselung können die selbstgekräuselten Fasern eine mechanische Kräuselung aufweisen. Das Versehen der Fasern mit der mechanischen Kräuselung erfolgt dabei bevorzugt während der Herstellung der Fasern, üblicherweise unter Verwendung von Stopfbuchsen oder von Sägezahnrädern. Diese mechanische Kräuselung dient der besseren Verarbeitungsfähigkeit der latent kräuselnden Fasern bei der Vliesstoffherstellung.

Erfindungsgemäß bevorzugt sind sowohl die selbstgekräuselten Fasern als auch die superabsorbierenden Fasern Stapelfasern. Dies ist vorteilhaft, da Nadelvliesstoffe mit hohem Volumen hergestellt werden können, welches dazu beiträgt, dem Nadelvliesstoff absorbierende Eigenschaften zu verleihen. Dabei beträgt die Stapelfaserlänge der selbstgekräuselten Fasern und/oder der superabsorbierenden Fasern unabhängig voneinander vorzugsweise zwischen 32 und 80 mm, bevorzugt zwischen 38 und 70 mm, und besonders bevorzugt von 40 bis 60 mm. Der Titer der selbstgekräuselten Fasern und/oder der superabsorbierenden Fasern beträgt unabhängig voneinander vorzugsweise zwischen 1,7 und 20 dtex, besonders bevorzugt zwischen 2,0 und 10 dtex.

Unter superabsorbierenden Fasern sind Fasern zu verstehen, die aus einem Material gebildet sind, das die Eigenschaft hat, wässrige Flüssigkeiten unter Bildung eines Hydrogels zu absorbieren. Bevorzugte superabsorbierende Fasern weisen eine freie Absorptionskapazität von wässriger NaCl-Lösung (0,9 Gew.%), gemessen wie in dem Abschnitt Messmethoden definiert, von mindestens 20 g/g, beispielsweise 20 g/g bis 70 g/g, bevorzugt von mindestens 30 g/g bis 60 g/g, besonders bevorzugt von 40 g/g bis 50 g/g auf.

Bevorzugte superabsorbierende Fasern enthalten Polymere, die ausgewählt sind aus der Gruppe bestehend aus Polyacrylsäure und/oder Polymethacrylsäure, Pfropf-Copolymeren ungesättigter Carbonsäuren, modifizierter Cellulose, aus gelbildenden Polysacchariden ausgenommen modifizierte Cellulose, sowie Gemischen hiervon.

Bevorzugte Polyacrylsäuren und/oder Polymethacrylsäuren sind ausgewählt aus Acrylsäurepolymeren, Methacrylsäurepolymeren, Acrylsäure/Methacrylsäure-Copolymeren und deren Gemischen. Dabei sind die Säuregruppen der Polymere bevorzugt teilweise zu Natriumsalzen neutralisiert. Ebenfalls bevorzugt besteht eine Vernetzung zwischen den Polymerketten. Erfindungsgemäß verwendbare superabsorbierende Fasern sind beispielsweise unter dem Handelsnahmen OASIS SAF, hergestellt von TECHNICAL ABSORBENTS LTD., erhältlich.

In einer weiteren bevorzugten Ausführungsform weisen die superabsorbierenden Fasern, insbesondere die Polyacrylat enthaltenden superabsorbierenden Fasern, eine zweischichtige Struktur auf, die eine äußere superabsorbierende Schicht, die mittels einer hydrolysierenden Behandlung mit Carboxylgruppen versehen wurde, und eine innere Schicht aus Polyacrylnitril aufweist. Solche Fasern sind in US4366206A beschrieben und sind unter dem Handelsnahmen LANSEAL, hergestellt von JAPAN EXLAN CO., LTD. erhältlich.

Ebenfalls geeignete superabsorbierende Fasern weisen eine Verbindung aus dem Copolymerisat von ungesättigten Carbonsäuren und aus Disacchariden, Oligosacchariden und/oder Polysacchariden auf. Solche Fasern sind beispielsweise in US4788237A beschrieben.

Bevorzugte Pfropf-Copolymer ungesättigter Carbonsäuren sind Copolymerisate von ungesättigten Carbonsäuren und aus Disacchariden, Oligosacchariden und/oder Polysacchariden.

Ebenfalls geeignete superabsorbierende Fasern werden durch die chemische Modifikation von cellulosischen Fasern hergestellt. Bevorzugte cellulosische Fasern sind carboxymethylierte cellulosische Fasern, insbesondere cellulosische Fasern, wie beschrieben in der EP0680344. Ebenfalls bevorzugte cellulosische Fasern sind carboxyethylierte Cellulosefasern, insbesondere Fasern, wie beschrieben in der WO2011022740A1. Ebenfalls bevorzugte cellulosische Fasern sind durch Alkylsulfonierung von cellulosischen Fasern erhaltene Fasern, insbesondere Fasern, wie beschrieben in der EP2196224A1.

Bevorzugte modifizierte Cellulose ist ausgewählt aus carboxymethylierter Cellulose, carboxyethylierter Cellulose, alkylsulfonierter Cellulose und deren Gemischen.

Ebenfalls geeignete superabsorbierende Fasern enthalten Polymere, die ausgewählt sind aus gelbildenden Polysacchariden, insbesondere aus Alginat, Pectin, Chitosan oder Hyaluronsäure und deren Gemischen. Besonders geeignete superabsorbierende Fasern dieser Art sind beschrieben in der EP1085912A1.

Neben den superabsorbierenden Fasern und den selbstgekräuselten Fasern kann der Nadelvliesstoff noch weitere Fasern enthalten, vorzugsweise ausgewählt aus Fasern, die eines oder mehrere der folgenden Faserrohmaterialien enthalten: Polyolefine, Cellulose, regenerierte Cellulose, insbesondere Viskose, Polyamide, Polyacrylnitrile, Elastane, Polyvinylchloride, Polylactide, Polyglykolide, Polyesteramide, Polycaprolactone, Polyhexamethylenterephthalate, Polyhydroxybutyrate, Polyhydroxyvalerate, tierische und/oder pflanzliche Naturfasern und/oder Polyester.

Bevorzugte weitere Fasern sind hydrophile Fasern. Erfindungsgemäß wird unter dem Begriff "hydrophile Fasern" verstanden, dass die betreffende Faser an der Oberfläche ein Fasermaterial aufweist, welches eine Oberflächenenergie, gemessen nach DIN 55660-2:2011-12 von > 35 mN/m aufweist. Besonders bevorzugte hydrophile Fasern sind ausgewählt aus hydrophilen Fasern, die ein oder mehrere Faserrohmaterialien enthalten, ausgewählt aus Cellulose, vorzugsweise regenerierter Cellulose, insbesondere Viskose.

Vorteilhaft an hydrophilen Fasern ist, dass ihre Verwendung zu einer besonders guten Benetzbarkeit des Nadelvliesstoffs auch nach thermischer Behandlung führt. Dies ist wiederum vorteilhaft für den vertikalen Flüssigkeitstransport.

Sofern vorhanden, liegen die weiteren Fasern vorzugsweise in einem Anteil von 5 Gew.% bis 30 Gew.%, noch bevorzugter von 10 Gew.% bis 20 Gew.%, bezogen auf das Gesamtgewicht des Nadelvliesstoffs, vor.

Erfindungsgemäß umfasst die Wundauflage einen Nadelvliesstoff. Unter Vliesstoffen werden textile Flächengebilde verstanden, die aus Fasern, Filamenten oder geschnittenen Garnen zu einem Faserflor geformt und verfestigt worden sind, mit einem Verfahren, das nicht Weben, Wirken oder Stricken ist.

Die Abgrenzung von Vliesstoffen gegenüber anderen textilen Flächengebilden wird in der Norm DIN EN ISO 9092:2019-08 definiert. Die Formung von Fasern zu einem Faserflor kann erfolgen durch verschiedene technische Verfahren, wie Krempeln, dem Airlaidverfahren, dem Nassvliesverfahren oder durch Schmelzspinnen. Bevorzugt sind gekrempelte Faserflore. Faserflore können zur besseren Verarbeitung auch vorverfestigt sein.

In einer bevorzugten Ausführungsform der Erfindung sind die superabsorbierenden Fasern und die selbstgekräuselten Fasern, vorzugsweise homogen, im Nadelvliesstoff verteilt. Dies bedeutet, dass die Fasern nicht in Form unterschiedlicher Lagen im Vliesstoff angeordnet sind. Eine solche homogene Verteilung kann, wie dem Fachmann bekannt ist, beispielsweise durch Krempeln der Fasern bei der Herstellung des Faserflors bewirkt werden. Die homogene Verteilung hat den Vorteil, dass die superabsorbierenden Fasern besonders gut von den selbstgekräuselten Fasern in ihre Gerüststruktur eingebunden werden können.

Der erfindungsgemäße Vliesstoff ist ein Nadelvliesstoff. Unter Nadelvliesstoffen sind Flächengebilde zu verstehen, die aus der Verfestigung eines Faserflors durch Nadeln gebildet werden. Die hierfür eingesetzten Nadeln verfügen über Spitzen, die im Faserflor einen vertikalen Einstichkanal erzeugen, sowie über Kerben, welche Fasern oder Faserbündel erfassen und bei Einstichbewegung in den vertikalen Einstichkanal hineinziehen. Dadurch weisen Nadelvliesstoffe Regionen hoher Faserdichte und teilweise vertikaler Orientierung der Fasern, sowie Regionen geringerer Faserdichte zwischen den Einstichkanälen auf. Nadelvliesstoffe haben den Vorteil, dass die Vernadelung Zonen schafft, in denen der Nadelvliesstoff fixiert ist, und die Faser zwischen den fixierten Zonen durch Kräuselung zusammengezogen wird. Dies führt zu einer hohen elastischen Verformbarkeit. Vorzugsweise weist der Nadelvliesstoff eine Nadeldichte von 50 bis 150 Einstichen/cm$^2$ auf, bevorzugt von 70 bis 120 Einstichen/cm$^2$, insbesondere von 90 bis 110 Einstichen/cm$^2$.

Der Nadelvliesstoff ist, wie oben dargelegt, vorzugsweise thermisch behandelt. Hierdurch kann die latente Kräuselungsfähigkeit der als Ausgangsmaterial verwendeten selbstkräuselnden Fasern ausgelöst werden. In der Regel ist der Nadelvliesstoff bei Temperaturen von 160°C bis 200°C, bevorzugt von 180°C bis 190°C thermisch behandelt.

In einer weiteren bevorzugten Ausführungsform der Erfindung weist die Wundauflage mindestens eine weitere Faserlage auf, die auf mindestens einer Seite des Nadelvliesstoffes angeordnet ist. Bevorzugt ist die weitere Faserlage durch Vernadeln mit dem Nadelvliesstoff verbunden. Dies ist vorteilhaft, weil durch die Befestigung durch Vernadelung Einstichkanäle gebildet werden, die einen beschleunigten Flüssigkeitstransport von der weiteren Faserlage in den Nadelvliesstoff ermöglichen. Bevorzugt ist die weitere Faserlage als Vliesstoff ausgebildet.

Besonders bevorzugt ist die mindestens eine weitere Faserlage dadurch mit dem Nadelvliesstoff verbunden, dass auf den als Ausgangsmaterial zur Herstellung des Nadelvliesstoffs verwendeten Faserflor mindestens ein weiterer Faserflor aufgebracht wurde und die Faserflore gemeinsam vernadelt wurden.

Sofern vorhanden, ist die mindestens eine weitere Faserlage in einer Ausführungsform hydrophil ausgebildet. Erfindungsgemäß wird unter dem Begriff "hydrophil ausgebildet" verstanden, dass die Faserlage eine Oberflächenenergie, gemessen nach DIN 55660-2:2011-12, von > 35 mN/m aufweist.

Dies ist vorteilhaft, weil eine solche Faserlage Flüssigkeit besonders schnell aufnimmt und in das Innere des Vliesstoffs weiterleitet.

In einer weiteren Ausführungsform ist die mindestens eine weitere Faserlage hydrophob ausgebildet. Erfindungsgemäß wird unter dem Begriff "hydrophobe Faserlage" verstanden, dass die Faserlage eine Oberflächenenergie, gemessen nach DIN 55660-2:2011-12, von < 35 mN/m aufweist. Das ist vorteilhaft, weil eine solche Faserlage in direktem Kontakt zu einer Wunde nicht zu Verkleben der Wundauflage mit der Wunde führt.

In einer weiteren bevorzugten Ausführungsform ist die mindestens eine weitere Faserlage thermoplastisch ausgebildet. Dies ist vorteilhaft, weil sich eine solche Faserlage glätten lässt, wodurch lose Faserenden eingebunden werden können. Dies ist zusätzlich vorteilhaft, weil eine solche Faserlage die thermische Laminierung mit weiteren üblichen Komponenten einer Wundauflage ermöglicht.

In einer weiteren bevorzugten Ausführungsform ist die mindestens eine weitere Faserlage thermoplastisch elastomer ausgebildet. Dies ist vorteilhaft, weil sich die Verwendung einer solchen Faserlage vorteilhaft auf die thermoplastischen und elastischen Eigenschaften der Wundauflage auswirkt.

In einer weiteren bevorzugten Ausführungsform weist die mindestens eine weitere Faserlage Fasern mit hydrophilen Eigenschaften in Kombination mit Fasern auf, die bei der Temperatur, bei der die Kräuselung der selbstgekräuselten Fasern ausgelöst wurde, thermoplastisches Verhalten zeigen. Dies ist von Vorteil, weil die so ausgebildete Faserlage wässrige Flüssigkeiten weiterleiten und flächig verteilen kann, bevor diese von der superabsorbierende Fasern enthaltenden Lage aufgenommen wird. Dies begünstigt die schnelle Aufnahme großer Mengen von Wundexsudat.

In einer weiteren bevorzugten Ausführungsform der Erfindung ist die Wundauflage, insbesondere der Nadelvliesstoff, mit mindestens einem antimikrobiellen Wirkstoff, beispielsweise ausgewählt aus der Gruppe bestehend aus Silber, Silbersalzen wie beispielsweise Silbernitrat oder Silbersulfat, Iod enthaltenden Verbindungen, Biguanidinen, beispielsweise Chlorhexidin oder Polyhexamethylenbiguanidin ("PHMB"), quartären Ammoniumverbindungen, beispielsweise Benzalkoniumchlorid, oder Octenidin Hydrochlorid und Gemischen hiervon ausgerüstet. Wenn vorhanden, weist die Wundauflage, insbesondere der Nadelvliesstoff den antimikrobiellen Wirkstoff vorzugsweise in einer Menge von 0,1 g bis 10 g pro Quadratmeter, vorzugsweise 0,5 g bis 5 g pro Quadratmeter auf.

Des Weiteren kann die die Wundauflage, insbesondere der Nadelvliesstoff, verschiedene Additive aufweisen. Bevorzugte Additive sind ausgewählt aus pharmakologischen Wirkstoffen oder Medikamenten, insbesondere Analgetika, entzündungshemmenden Mitteln, wundheilungsfördernden Mitteln, blutstillenden Mitteln, Enzymen, Aminosäuren, Antioxidantien, Peptiden, Peptidsequenzen, Polysacchariden, insbesondere Chitosan, Wachstumsfaktoren, insbesondere Purinen, Pyrimidinen, geruchsadsorbierenden Additiven, insbesondere Aktivkohle und/oder Verarbeitungshilfsstoffen, insbesondere oberflächenaktiven Substanzen, Netzmitteln, Avivagen, Antistatika und/oder Gemischen hiervon.

Wenn vorhanden, weist die die Wundauflage, insbesondere der Nadelvliesstoff die weiteren Additive in einer Menge von 0,1 g bis 10 g pro Quadratmeter, vorzugsweise 0,5 g bis 5 g pro Quadratmeter auf.

Des Weiteren kann die Wundauflage weitere übliche Komponenten enthalten, wie hydrophile Schäume, beispielsweise Polyurethanschäume, flüssigkeitsundurchlässige Rückenlagen und/oder Klebemassen.

Die erfindungsgemäße Wundauflage und/oder der Nadelvliesstoff zeichnet sich aus durch einen geringen Schrumpf bei Kontakt mit Flüssigkeit oder mit Luftfeuchtigkeit während der Lagerung. Erfindungsgemäß bevorzugt weist die Wundauflage und/oder der Nadelvliesstoff einen Schrumpf, gemessen wie im Abschnitt Messmethoden definiert, nach 24 Stunden von 0% bis 10%, noch bevorzugter von 0% bis 5% auf.

Die erfindungsgemäße Wundauflage und/oder der Nadelvliesstoff zeichnet sich ferner durch eine hohe Absorption aus. Erfindungsgemäß bevorzugt weist die Wundauflage und/oder der Nadelvliesstoff eine Aufnahmekapazität, gemessen wie im Abschnitt Messmethoden definiert, für 0,9%ige, wässrige Natriumchlorid-Lösung von 1000% bis 5000%, oder von 1000% bis 2500% auf.

Die erfindungsgemäße Wundauflage und/oder der Nadelvliesstoff zeichnet sich durch eine hohe Elastizität aus. Bevorzugt beträgt der plastische Anteil der Verformung $\varepsilon(pl)$ der Wundauflage und/oder des Nadelvliesstoffs, bestimmt wie im Kapitel Messmethoden definiert, längs und/oder quer, besonders bevorzugt längs und quer, weniger als 12%, bevorzugt weniger als 10%, und besonders bevorzugt weniger als 8%.

Die erfindungsgemäße Wundauflage und/oder der Nadelvliesstoff zeichnet sich ferner durch gute Gelblockierungseigenschaften aus, was sich in einer niedrigen Steighöhe von NaCl-Lösung widerspiegelt. Bevorzugt beträgt die Steighöhe von 0,9 Gew.% NaCl-Lösung der erfindungsgemäßen Wundauflage und/oder des Nadelvliesstoffs, bestimmt wie im Kapitel Messmethoden definiert, längs und/oder quer, bevorzugt längs und quer, weniger als 30 mm, bevorzugt weniger als 20 mm.

Weiter bevorzugt ist die erfindungsgemäße Wundauflage und/oder der Nadelvliesstoff steril. Das bedeutet, dass die Wundauflage und/oder der Nadelvliesstoff, vorzugsweise mit einem Standard-Sterilisationsverfahren, sterilisiert wurde. Zweckmäßigerweise wurde die Wundauflage durch mindestens eine der folgenden Methoden sterilisiert: Hochenergiestrahlung, insbesondere $\gamma$-Strahlung, durch Hitze, unter Druck, durch Dampf, insbesondere in einem Autoklaven, oder durch reaktive Chemikalien, wie Ethylenoxid.

Weiter bevorzugt ist die erfindungsgemäße Wundauflage und/oder der Nadelvliesstoff kalandriert.

Die erfindungsgemäße Wundauflage kann vorteilhafter Weise mit einem Verfahren hergestellt werden, umfassend die folgenden Schritte:

1) Herstellung und/oder Bereitstellung eines Faserflors, der 30 Gew.% bis 80 Gew.%, bevorzugt 40 Gew.% bis 75 Gew.%, noch bevorzugter 45 Gew.% bis 70 Gew.%, insbesondere 55 Gew.% bis 65 Gew.%, bezogen auf das Gesamtgewicht des Faserflors, superabsorbierende Fasern, und 70 Gew.% bis 20 Gew.%, bevorzugt 60 Gew.% bis 25 Gew.%, noch bevorzugter 55 Gew.% bis 30 Gew.%, insbesondere 45 Gew.% bis 35 Gew.% bezogen auf das Gesamtgewicht des Faserflors, selbstkräuselnde Fasern, enthält;
2) Verfestigung des in Schritt 1) erhaltenen Faserflors zu einem Nadelvliesstoff durch Vernadeln;
3) Auslösen der Selbstkräuselung der selbstkräuselnden Fasern des in Schritt 2) erhaltenen verfestigten Faserflors, vorzugsweise durch thermisches Behandeln, wobei eine Wundauflage erhalten wird.

Die Verfahrensschritte werden vorzugsweise nacheinander durchgeführt.

Bevorzugte Ausführungsformen des erfindungsgemäßen Verfahrens umfassen die bevorzugten Ausführungsformen, die in Bezug auf die erfindungsgemäße Wundauflage beschrieben sind.

Bevorzugt erfolgt die Herstellung des Faserflors in Schritt 1) derart, dass eine homogene Verteilung der Faserarten erfolgt. Dies kann beispielsweise durch Krempeln erzielt werden. Entsprechend wird bevorzugt ein gekrempelter Faserflor bereitgestellt.

In einer weiteren bevorzugten Ausführungsform wird der Faserflor mit einem Querleger abgelegt.

Werden weitere Fasern verwendet, so werden diese bei der Herstellung des Faserflors vorzugsweise homogen mit den superabsorbierenden Fasern und den selbstkräuselnden Fasern vermischt.

Als ein Ausgangsmaterial zur Herstellung des Faserflors werden selbstkräuselnde Fasern eingesetzt. Unabhängig von ihrer Selbstkräuselungsfähigkeit können die selbstkräuselnden Fasern eine mechanische Kräuselung aufweisen. Die Ausformung der mechanischen Kräuselung erfolgt dabei bevorzugt während der Herstellung der Fasern, üblicherweise unter Verwendung von Stopfbuchsen oder von Sägezahnrädern. Diese mechanische Kräuselung ist vorteilhaft, weil sie zu einer besseren Verarbeitungsfähigkeit der selbstkräuselnden Fasern führt.

In Schritt 2) erfolgt die Verfestigung des in Schritt 1) erhaltenen Faserflors zu einem Nadelvliesstoff durch Vernadeln. Vorzugsweise erfolgt die Vernadelung mit 50 bis 150 Einstichen/cm$^2$, bevorzugt mit 70 bis 120 Einstichen/cm$^2$, insbesondere mit 90 bis 110 Einstichen/cm$^2$.

In Schritt 3) erfolgt das Auslösen der Selbstkräuselung der selbstkräuselnden Fasern des in Schritt 2) erhaltenen verfestigten Faserflors, vorzugsweise durch thermisches Behandeln. In der Regel erfolgt das Erhitzen bei Temperaturen von 160°C bis 200°C, bevorzugt von 180°C bis 190°C.

Vorzugsweise wird die Selbstkräuselung der selbstkräuselnden Faser ausgelöst durch Behandlung des Nadelvliesstoffs in einem Durchlauftrockner, der mit einem Ofenband ausgerüstet ist, unter Voreilung mit Durchhang in Maschinenrichtung. Dies ist von Vorteil, weil der auf diese Weise erzeugte Nadelvliesstoff gleichmäßige Elastizität in Längs- und in Querrichtung zeigt.

In einer weiteren bevorzugten Ausführungsform wird die Selbstkräuselung der selbstkräuselnden Faser ausgelöst durch Behandlung des Nadelvliesstoffs in einem Durchlauftrockner mit einem Spannrahmen. Bevorzugt wird dabei die Breite des eingespannten Materials durch Zusammenführen des Spannrahmens während der Behandlung verringert. Dies ist von Vorteil, weil der auf diese Weise erzeugte Nadelvliesstoff selektive Elastizität in Querrichtung zeigt. Dies ergibt eine gute Verarbeitungsfähigkeit auf Konfektionieranlagen für Wundauflagen.

In einer weiteren bevorzugten Ausführungsform wird die Selbstkräuselung der selbstkräuselnden Fasern ausgelöst durch Behandlung des Nadelvliesstoffs in einem Durchlauftrockner mit einem Spannrahmen, wobei die Breite des eingespannten Materials durch Zusammenführen des Spannrahmens während der Behandlung verringert wird, und gleichzeitig der Abstand zwischen den Befestigungspunkten des Nadelvliesstoffs am Spannrahmens durch Zusammenführen der einzelnen Befestigungselemente des Spannrahmens in Maschinenlaufrichtung verringert wird.

Dies ist von Vorteil, weil der auf diese Weise erzeugte Nadelvliesstoff unterschiedlich starke Elastizität in Längs- und Quer-Richtung zeigt.

Des Weiteren kann ein Konfektionieren und/oder Nachbehandeln der einzelnen Komponenten des Verfahrens, insbesondere der Wundauflage und/oder des Nadelvliesstoffs durchgeführt werden.

Ein bevorzugtes Nachbehandeln umfasst die Aufbringung eines Netzmittels. Zweckmäßigerweise wird das Netzmittel auf die Wundauflage und/oder den Nadelvliesstoff aufgebracht.

Ein weiteres bevorzugtes Nachbehandeln umfasst die Sterilisierung. Zweckmäßigerweise wird dabei die Wundauflage und/oder der Nadelvliesstoff sterilisiert, insbesondere durch mindestens eine der folgenden Methoden: Hochenergiestrahlung, insbesondere γ-Strahlung, durch Hitze, unter Druck, durch Dampf, insbesondere in einem Autoklaven, oder durch reaktive Chemikalien, wie Ethylenoxid.

Ein weiteres bevorzugtes Nachbehandeln umfasst eine Kalandrierung. Dabei wird vorzugsweise die Wundauflage und/oder der Nadelvliesstoff kalandriert. Bevorzugt erfolgt die Kalandrierung mit mindestens einer Kalanderwalze mit strukturierter Oberfläche. Dies ist von Vorteil, weil eine zusätzliche Verfestigung erhalten werden und/oder eine strukturierte Oberfläche geschaffen werden kann.

Ein ebenfalls denkbares Nachbehandeln ist eine Nachverfestigung, eine Beschichtung und/oder eine chemische Ausrüstung, vorzugsweise der Wundauflage und/oder des Nadelvliesstoffs.

Ein ebenfalls denkbares Nachbehandeln ist ein Versehen mit Verarbeitungshilfsstoffen und/oder Additiven. Sofern eine thermische Behandlung in Schritt 3) durchgeführt wird, erfolgt dieses Nachbehandeln vorzugsweise nach der thermischen Behandlung. Als Verarbeitungshilfsstoffe können beispielsweise Avivage, antistatische Mittel, Tenside, Stabilisatoren und/oder Gleitmittel zum Einsatz kommen.

Ein weiterer Gegenstand der Erfindung ist die Verwendung eines Nadelvliesstoffs, enthaltend:

- 30 Gew.% bis 80 Gew.%, bevorzugt 40 Gew.% bis 75 Gew.%, noch bevorzugter 45 Gew.% bis 70 Gew.%, insbesondere 55 Gew.% bis 65 Gew.%, bezogen auf das Gesamtgewicht des Nadelvliesstoffs, superabsorbierende Fasern;
- 70 Gew.% bis 20 Gew.%, bevorzugt 60 Gew.% bis 25 Gew.%, noch bevorzugter 55 Gew.% bis 30 Gew.%, insbesondere 45 Gew.% bis 35 Gew.% bezogen auf das Gesamtgewicht des Nadelvliesstoffs, selbstgekräuselte Fasern,

zur Herstellung einer Wundauflage.

Bevorzugte Ausführungsformen der erfindungsgemäßen Verwendung umfassen die bevorzugten Ausführungsformen, die in Bezug auf die erfindungsgemäße Wundauflage beschrieben sind, mutatis mutandis.

Im Folgenden wird die Erfindung anhand mehrerer nicht beschränkender Beispiele näher erläutert.

**Ausführungsbeispiel 1**

An einer Krempelmaschine mit Querleger wurden drei Faserflore erzeugt mit einem Flächengewicht von 150 g/m$^2$ mit unterschiedlichen Mischungsverhältnissen von superabsorbierender Faser (Oasis 112) und latent selbstkräuselnder Faser (Beispiele 1 bis 3), sowie ein weiterer Faserflor nur aus latent selbstkräuselnder Polyesterfaser (Vergleichsbeispiel 1). Die Faserflore wurden verfestigt durch Vernadelung mit einem Nadelstuhl, bestückt mit Nadeln von Hersteller Groz-Beckert von der Type R222 G, mit einer Einstichtiefe von 10 mm und 100 Einstichen/cm$^2$. Die auf diese Art erhaltenen Nadelvliesstoffe wurden bei 185°C mit 5%iger Voreilung durch einen Durchlauftrockner geführt, so dass das Material durch Auslösung der latenten Selbstkräuselung in Querrichtung frei schrumpfen konnte.

Als Referenz-Probe mit superabsorbierender Faser diente ein Nadelvliesstoff aus 60 Gew.% superabsorbierender Faser (Oasis 112) und 40 Gew.% einer mechanisch gekräuselten Polyesterfaser, die nicht über Selbstkräuselung verfügt (Vergleichsbeispiel 2).

|  | Superabsorbierende Faser | Selbstkräuselnde Faser |
|---|---|---|
| Beispiel 1 | 20 Gew.% | 80 Gew.% |
| Beispiel 2 | 40 Gew.% | 60 Gew.% |
| Beispiel 3 | 60 Gew.% | 40 Gew.% |
| Vergleichsbeispiel 1 | 0 Gew.% | 100 Gew.% |
| Vergleichsbeispiel 2 | 60 Gew.% | 0 Gew.% |

Von den so hergestellten Nadelvliesstoffen wurde die Elastizität und das Aufnahmevermögen von 0,9%iger Natriumchloridlösung bestimmt.

|  | Absorption | $\varepsilon(pl)$ quer |
|---|---|---|
| Beispiel 1 | 570% | 6,0% |
| Beispiel 2 | 1120% | 7,0% |
| Beispiel 3 | 1890% | 7,7% |
| Vergleichsbeispiel 1 | 460% | 4,9% |
| Vergleichsbeispiel 2 | 2100% | 15,9% |

Es konnte gezeigt werden, dass alle erfindungsgemäßen Beispiele eine hohe elastische Rückerholung in Kombination mit einer hohen Absorption von NaCl-Lösung aufweisen.

Von den so hergestellten Nadelvliesstoffen, Beispiel 3 sowie Vergleichsbeispiel 2 wurde der Schrumpf bei Kontakt mit Luftfeuchtigkeit bestimmt, wie im Kapitel Messmethoden beschrieben.

|  | 2 h | 4 h | 6 h | 24 h |
|---|---|---|---|---|
| Beispiel 3 | 0% | 1,8% | 2,0% | 3,3% |
| Vergleichsbeispiel 2 | 2,0% | 6,0% | 8,7% | 11,3% |

Es konnte gezeigt werden, dass Beispiel 3 einen signifikant geringeren Schrumpf zeigt, als Vergleichsbeispiel 2, welches den gleichen Anteil von superabsorbierenden Fasern (Oasis 112) enthält.

**Ausführungsbeispiel 2**

Ein weiterer Nadelvliesstoff wurde hergestellt mit der gleichen Methode wie Beispiel 3, mit 60 Gew% der superabsorbierenden Faser Lanseal FK.

|  | Superabsorbierende Faser | Selbstkräuselnde Faser |
|---|---|---|
| Beispiel 4 | 60% Lanseal | 40% |

Von Beispiel 3, Beispiel 4 sowie von Vergleichsbeispiel 2 wurde die Steighöhe von NaCl-Lösung in Längs- und Querrichtung bestimmt.

|  | Steighöhe in mm (längs) | Steighöhe in mm (quer) |
|---|---|---|
| Beispiel 3 | 19 | 22 |
| Beispiel 4 | 14 | 15 |
| Vergleichsbeispiel 2 | 34 | 34 |

Es konnte gezeigt werden, dass sowohl Beispiel 3 als auch Beispiel 4 eine signifikant geringere Steighöhe aufweisen als Vergleichsbeispiel 2, welches den gleichen Anteil von superabsorbierenden Fasern (Oasis 112) enthält.

**Ausführungsbeispiel 3**

Es wurde die Einbindung des durch die superabsorbierenden Fasern gebildeten Gels von Beispiel 3 und Vergleichsbeispiel 2 verglichen. Hierzu wurde jeweils ein 10 x 10 cm großes Prüfstück ausgestanzt, für 5 min in deionisiertes Wasser eingelegt, anschließend für 5 min auf einem Gitter mit einem Gewichtstück mit einer Grundfläche von 10 x 10 cm und einem Gewicht von 4100 kg beaufschlagt, und danach auf einem Klebeband (CMC 10966 Polyester Tape des Herstellers CMC Klebetechnik) aufgelegt und zweimal mit einer Walze mit einem Gewicht von 1580 kg Gewicht angedrückt. Zuletzt wurden die Prüfstücke wieder von dem Klebeband abgezogen. Das aus dem gelierten Nadelvliesstoff ausgetretene Gel wurde händisch beurteilt, und das Erscheinungsbild der Prüfstücke verglichen.

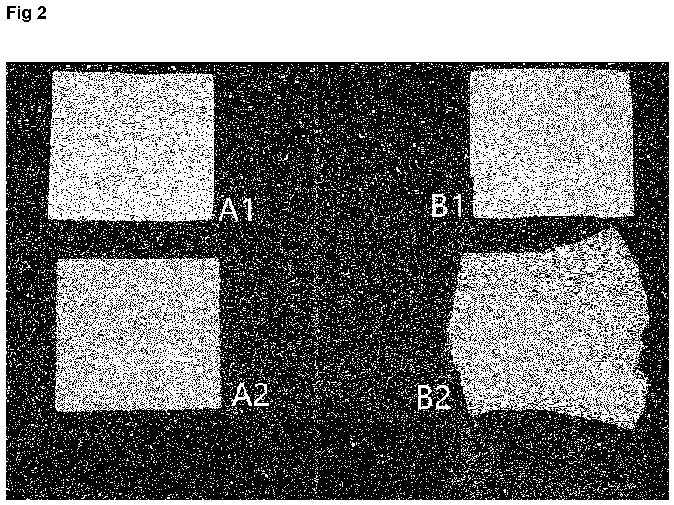

Bei Beispiel 3 trat signifikant weniger fühlbares Gel aus der Oberfläche aus als bei Vergleichsbeispiel 2. Zudem blieb bei Beispiel 3 nach Abzug vom Klebeband die Form wesentlich besser erhalten, wie in Fig. 2 gezeigt (A1: Beispiel 3 vor der Prüfung; A2: Beispiel nach der Prüfung; B1: Vergleichsbeispiel 2 vor der Prüfung; B2: Vergleichsbeispiel 2 nach der Prüfung).

**Messmethoden**

**Freie Absorptionskapazität von Fasern:**

1 g der Superabsorber-Faserprobe wird eingewogen ($W_1$) in einen Teebeutel (Acryl/Polyester-Gaze mit feinen Maschen) gelegt und der Beutel wird eine Stunde lang in eine überschüssige Menge NaCl-Lösung 0,9 Gew.% (temperiert auf 20°C) getaucht. Dann wird die überschüssige Lösung durch Aufhängen des Beutels entfernt, bis keine Flüssigkeit mehr abtropft. Der Teebeutel wird gewogen (W2). Das Verfahren wird mit einem leeren Teebeutel wiederholt und gewogen (W0) und die Quellfähigkeit wird berechnet nach folgender Gleichung:

$$\text{Freie Absorptionskapazität} = (W2-W1-W0)/W1$$

**Schrumpf bei Kontakt mit Luftfeuchtigkeit:**

Der Schrumpf wird bestimmt durch Ausstanzen von 10,0 cm x 10,0 cm (Fläche1) großen Stücken aus der Wundauflage und/oder dem Vliesstoff, und Lagerung in einem Klimaschrank bei einer Temperatur von 37°C und einer relativen Luftfeuchtigkeit von 45%. Die ausgestanzten und gelagerten Stücke werden nach Ablauf einer festgelegten Zeit aus dem Klimaschrank entnommen, und die Größe der Stücke abgemessen (Fläche 2). Der Schrumpf der ausgestanzten Stücke kann dann nach folgender Formel berechnet werden:

$$Schrumpf\ [\%] = 100 - \frac{Fläche2\ [cm^2]}{Fläche1\ [cm^2]} * 100$$

**Aufnahmekapazität:**

Die Aufnahmekapazität wird bestimmt durch Ausstanzen und Auswiegen (Gewicht 1) von 10,0 cm x 10,0 cm großen Stücken aus der Wundauflage und/oder dem Vliesstoff und Eintauchen in eine 0,9 %ige wässrige Natriumchlorid-Lösung (temperiert auf 20°C). Die ausgestanzten und getränkten Stücke werden aus der Lösung entnommen und für 2min abgetropft. Danach werden sie erneut ausgewogen (Gewicht 2). Die Aufnahmekapazität wird dann nach folgender Formel berechnet:

$$Aufnahmekapazit\ddot{a}t\ [\%] = \frac{Gewicht\ 2\ [g]\ -\ Gewicht\ 1\ [g]}{Gewicht\ 1\ [g]} * 100$$

**Elastizität:**

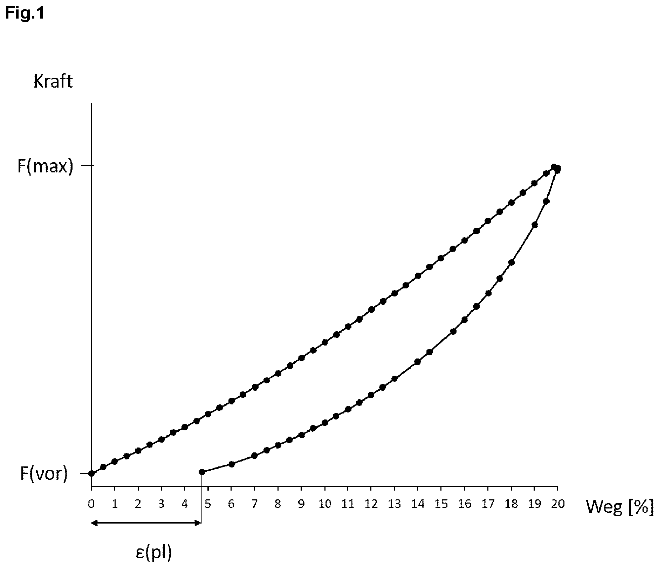

Zur Charakterisierung der Elastizität wird aus der Wundauflage und/oder dem Vliesstoff ein rechteckiger 50 mm breiter Prüfkörper ausgestanzt und eingespannt mit einer Einspannlänge von 200 mm in eine Zugprüfmaschine (zum Beispiel Typ Z020/SN3A.02SO1 vom Hersteller Zwick/Roell). Der Prüfkörper wird mit einer Geschwindigkeit von 10 mm/min gezogen bis eine Vorkraft F(vor) von 0,2 N gemessen wird. Die Länge bei Erreichen von F(vor) wird als Ausgangslänge L(0) betrachtet. Ab dann wird mit einer konstanten Geschwindigkeit von 100 mm/min weiter gezogen auf eine Strecke von L(0) * 120%, und anschließend wieder mit 100 mm/min zurückgeführt, bis die gemessene Kraft wieder auf 0,2 N abgefallen ist. Die zugehörige Strecke relativ zu L(0) im Spannungs-Dehnungsdiagramm wird interpretiert als plastischer Anteil der Verformung ε(pl). Eine Wundauflage und/oder ein Vliesstoff mit hoher Elastizität ist charakterisiert durch einen geringen Wert von ε(pl). Alle Messungen werden 5fach ausgeführt, und die Ergebnisse gemittelt.
Ein beispielhaftes Spannungs-Dehnungsdiagramm zur Illustration ist in Fig. 1 gezeigt.

**Steighöhe NaCl-Lösung**

Es wird die Steighöhe NaCl 0,9 Gew.% in Anlehnung an DIN EN ISO 9073-06 unter Verwendung einer Apparatur bestehend aus einer Flüssigkeitswanne, vertikalen Maßstäben mit einer Skalenteilung von 1 mm-Intervallen, sowie Einspannklemmen, deren Höhe relativ zur Flüssigkeitswanne verstellt werden kann. Die Kunststoffwanne wird bis auf Höhe des Überlaufs mit 0,9 Gew%. NaCl-Lösung gefüllt, und der Nullpunkt der Maßstäbe wird auf die Füllhöhe der NaCl-Lösung justiert.
Ein Prüfkörper von 250 mm x 30 mm wird ausgestanzt. Ein Ende des Prüfkörpers wird mit zwei Löchern versehen und mit einem Metallstab, der durch die Löcher geführt wird, beschwert. Der Probenkörper wird an der Einspannklemme befestigt, so dass das mit dem Metallstab beschwerte Ende nach unten zeigt und über der Flüssigkeitsoberfläche hängt.
Zu Beginn der Messung wird die Einstellklammer so weit abgesenkt, dass das mit dem Metallstab beschwerte Ende vollständig in die Flüssigkeit abgesenkt ist. Nach 5 Minuten wird die Höhe der Flüssigkeitsfront mit Hilfe der Maßstäbe abgelesen. Bei ungerader Flüssigkeitsfront wird der höchste Punkt der Steighöhe abgelesen.

**Patentansprüche**

1. Wundauflage umfassend einen Nadelvliesstoff enthaltend:

   - 30 Gew.% bis 80 Gew.%, bezogen auf das Gesamtgewicht des Nadelvliesstoffs, superabsorbierende Fasern;
   - 70 Gew.% bis 20 Gew.%, bezogen auf das Gesamtgewicht des Nadelvliesstoffs, selbstgekräuselte Fasern.

2. Wundauflage nach Anspruch 1, **dadurch gekennzeichnet, dass** der Nadelvliesstoff thermisch behandelt ist.

3. Wundauflage nach Anspruch 1 oder 2, **dadurch gekennzeichnet, dass** die selbstgekräuselten Fasern Fasermaterialien aufweisen, die ausgewählt sind aus der Gruppe der Polyamide, beispielsweise Polyamid 6, Polyamid 6,6 oder Polyamid 11, der Polyester, beispielsweise Polyethylenterephthalat (PET) oder Polybutylenterephthalat (PBT), Polybutylensuccinat (PBS) und der Polyolefine, beispielsweise Polypropylen (PP) oder Polyethylen (PE), oder aus deren Gemischen oder Copolymeren.

4. Wundauflage nach einem oder mehreren der vorangehenden Ansprüche, **dadurch gekennzeichnet, dass** die selbstgekräuselten Fasern hergestellt sind aus selbstkräuselnden Fasern, die mindestens zwei Komponenten aufweisen, wobei eine Komponente ein anderes Schrumpfverhalten unter thermischer Behandlung aufweist als die andere Komponente und wobei die Anordnung der Komponenten eine dreidimensionale Verformung der Faser unter thermischer Behandlung induziert und/oder dass die selbstgekräuselten Fasern hergestellt sind aus selbstkräuselnden Fasern, bei denen ein unterschiedliches Schrumpfverhalten unter thermischer Behandlung mindestens einer Faserkomponente gegenüber mindestens einer anderen Faserkomponente vorliegt.

**5.** Wundauflage nach einem oder mehreren der vorangehenden Ansprüche, **dadurch gekennzeichnet, dass** die selbstgekräuselten Fasern und/oder die als Ausgangsmaterial verwendeten selbstkräuselnden Fasern als Bikomponenten-Fasern vorliegen.

**6.** Wundauflage nach einem oder mehreren der vorangehenden Ansprüche, **dadurch gekennzeichnet, dass** die selbstgekräuselten Fasern und die superabsorbierenden Fasern Stapelfasern sind, wobei die Stapelfaserlänge der selbstgekräuselten Fasern und/oder der superabsorbierenden Fasern unabhängig voneinander vorzugsweise zwischen 32 und 80 mm beträgt und/oder der Titer der selbstgekräuselten Fasern und/oder der superabsorbierenden Fasern unabhängig voneinander vorzugsweise zwischen 1,7 und 20 dtex beträgt.

**7.** Wundauflage nach einem oder mehreren der vorangehenden Ansprüche, **dadurch gekennzeichnet, dass** die superabsorbierenden Fasern Polymere enthalten, die ausgewählt sind aus der Gruppe bestehend aus Polymeren ungesättigter Carbonsäuren, Pfropf-Copolymeren ungesättigter Carbonsäuren, modifizierter Cellulose, aus gelbildenden Polysacchariden ausgenommen modifizierte Cellulose, sowie Gemischen hiervon.

**8.** Wundauflage nach einem oder mehreren der vorangehenden Ansprüche, **dadurch gekennzeichnet, dass** der Nadelvliesstoff noch weitere Fasern enthält, vorzugsweise ausgewählt aus Fasern, die eines oder mehrere der folgenden Faserrohmaterialien enthalten: Polyolefine, Cellulose, regenerierte Cellulose, insbesondere Viskose, Polyamide, Polyacrylnitrile, Elastane, Polyvinylchloride, Polylactide, Polyglykolide, Polyesteramide, Polycaprolactone, Polyhexamethylenterephthalate, Polyhydroxybutyrate, Polyhydroxyvalerate, tierische und/oder pflanzliche Naturfasern und/oder Polyester.

**9.** Wundauflage nach Anspruch 8, **dadurch gekennzeichnet, dass** die weiteren Fasern hydrophile Fasern sind, bevorzugt ausgewählt aus hydrophilen Fasern, die ein oder mehrere Faserrohmaterialien enthalten, ausgewählt aus Cellulose, regenerierter Cellulose, insbesondere Viskose.

**10.** Wundauflage nach Anspruch 8 oder 9, **dadurch gekennzeichnet, dass** die weiteren Fasern in einem Anteil von 5 Gew.% bis 30 Gew.%, noch bevorzugter von 10 Gew.% bis 20 Gew.%, bezogen auf das Gesamtgewicht des Nadelvliesstoffs, vorliegen.

**11.** Wundauflage nach einem oder mehreren der vorangehenden Ansprüche, **dadurch gekennzeichnet, dass** der Nadelvliesstoff eine Nadeldichte von 50 bis 150 Einstichen/cm$^2$, bevorzugt von 70 bis 120 Einstichen/cm$^2$, insbesondere von 90 bis 110 Einstichen/cm$^2$ aufweist.

**12.** Wundauflage nach einem oder mehreren der vorangehenden Ansprüche, **dadurch gekennzeichnet, dass** die Wundauflage mindestens eine weitere Faserlage aufweist, die auf mindestens einer Seite des Nadelvliesstoffes angeordnet ist, wobei die mindestens eine weitere Faserlage vorzugsweise durch Vernadeln mit dem Nadelvliesstoff verbunden ist.

**13.** Wundauflage nach Anspruch 12, **dadurch gekennzeichnet, dass** die mindestens eine weitere Faserlage hydrophil ausgebildet ist.

**14.** Wundauflage nach einem oder mehreren der vorangehenden Ansprüche, **dadurch gekennzeichnet, dass** die Wundauflage und/oder der Nadelvliesstoff einen Schrumpf bei Kontakt mit Luftfeuchtigkeit, gemessen wie im Abschnitt Messmethoden definiert, nach 24 Stunden von 0% bis 10%, noch bevorzugter von 0% bis 5% aufweist und/oder der Nadelvliesstoff eine Aufnahmekapazität, gemessen wie im Abschnitt Messmethoden definiert, für 0,9% ige, wässrige Natriumchlorid-Lösung von 1000% bis 5000%, oder von 1000% bis 2500% aufweist und/oder der plastische Anteil der Verformung $\varepsilon(pl)$ der Wundauflage und/oder des Nadelvliesstoffs, bestimmt wie im Kapitel Messmethoden definiert, längs und/oder quer, besonders bevorzugt längs und quer, weniger als 12%, bevorzugt weniger als 10%, und besonders bevorzugt weniger als 8% beträgt und/oder die Steighöhe von 0,9 Gew.% NaCl-Lösung der Wundauflage und/oder des Nadelvliesstoffs, bestimmt wie im Kapitel Messmethoden definiert, längs und/oder quer, bevorzugt längs und quer, weniger als 30 mm, bevorzugt weniger als 20 mm beträgt.

**15.** Verfahren zur Herstellung einer Wundauflage, umfassend die folgenden Schritte:

1) Herstellung und/oder Bereitstellung eines Faserflors, der 30 Gew.% bis 80 Gew.%, bezogen auf das Gesamtgewicht des Faserflors, superabsorbierende Fasern und 70 Gew.% bis 20 Gew, bezogen auf das Gesamtgewicht des Faserflors, selbstkräuselnde Fasern, enthält;
2) Verfestigung des in Schritt 1) erhaltenen Faserflors zu einem Nadelvliesstoff durch Vernadeln;
3) Auslösen der Selbstkräuselung der selbstkräuselnden Fasern des in Schritt 2) erhaltenen verfestigten Faserflors, vorzugsweise durch thermisches Behandeln, wobei eine Wundauflage erhalten wird.

**16.** Verwendung eines Nadelvliesstoffs, enthaltend:

- 30 Gew.% bis 80 Gew.%, bezogen auf das Gesamtgewicht des Nadelvliesstoffs, superabsorbierende Fasern;
- 70 Gew.% bis 20 Gew, bezogen auf das Gesamtgewicht des Nadelvliesstoffs, selbstgekräuselte Fasern,

zur Herstellung einer Wundauflage.


**Claims**

**1.** Wound dressing comprising a needlefelt fabric containing:

- 30% to 80% by weight, based on the total weight of the needlefelt fabric, of superabsorbent fibres;
- 70% to 20% by weight, based on the total weight of the needlefelt fabric, of self-crimped fibres.

**2.** Wound dressing according to Claim 1, **characterized in that** the needlefelt fabric is thermally treated.

**3.** Wound dressing according to Claim 1 or 2, **characterized in that** the self-crimped fibres comprise fibre materials selected from the group of polyamides, for example polyamide 6, polyamide 6,6 or polyamide 11, polyesters, for example polyethylene terephthalate (PET) or polybutylene terephthalate (PBT), polybutylene succinate (PBS) and polyolefins, for example polypropylene (PP) or polyethylene (PE), or from mixtures or copolymers thereof.

**4.** Wound dressing according to one or more of the preceding claims, **characterized in that** the self-crimped fibres are produced from self-crimping fibres comprising at least two components, wherein one component has different shrinkage characteristics under thermal treatment than the other component and wherein the arrangement of the components induces a three-dimensional deformation of the fibres under thermal treatment and/or **in that** the self-crimped fibres are produced from self-crimping fibres for which there are different shrinkage characteristics under thermal treatment of at least one fibre component compared to at least one other fibre component.

**5.** Wound dressing according to one or more of the preceding claims, **characterized in that** the self-crimped fibres and/or the self-crimping fibres used as starting material are present as bicomponent fibres.

**6.** Wound dressing according to one or more of the preceding claims, **characterized in that** the self-crimped fibres and the superabsorbent fibres are staple fibres, wherein the staple fibre length of the self-crimped fibres and/or of the superabsorbent fibres independently of one another is preferably between 32 and 80 mm and/or the linear density of the self-crimped fibres and/or of the superabsorbent fibres independently of one another is preferably between 1.7 and 20 dtex.

**7.** Wound dressing according to one or more of the preceding claims, **characterized in that** the superabsorbent fibres contain polymers selected from the group consisting of polymers of unsaturated carboxylic acids, graft copolymers of unsaturated carboxylic acids, modified cellulose, of gel-forming polysaccharides excepting modified cellulose, and mixtures thereof.

**8.** Wound dressing according to one or more of the preceding claims, **characterized in that** the needlefelt fabric contains further fibres, preferably selected from fibres containing one or more of the following fibre raw materials: polyolefins, cellulose, regenerated cellulose, especially viscose, polyamides, polyacrylonitriles, elastanes, polyvinyl chlorides, polylactides, polyglycolides, polyesteramides, polycaprolactones, polyhexamethylene terephthalates, polyhydroxybutyrates, polyhydroxyvalerates, animal and/or vegetable natural fibres and/or polyesters.

9. Wound dressing according to Claim 8, **characterized in that** the further fibres are hydrophilic fibres, preferably selected from hydrophilic fibres containing one or more fibre raw materials selected from cellulose, regenerated cellulose, especially viscose.

10. Wound dressing according to Claim 8 or 9, **characterized in that** the further fibres are present in a fraction of 5% to 30% by weight, even more preferably of 10% to 20% by weight, based on the total weight of the needlefelt fabric.

11. Wound dressing according to one or more of the preceding claims, **characterized in that** the needlefelt fabric has a needle density of 50 to 150 stitches/cm$^2$, preferably of 70 to 120 stitches/cm$^2$, in particular of 90 to 110 stitches/cm$^2$.

12. Wound dressing according to one or more of the preceding claims, **characterized in that** the wound dressing has at least one further fibre layer which is arranged on at least one side of the needlefelt fabric, wherein the at least one further fibre layer is bonded to the needlefelt fabric preferably by needling.

13. Wound dressing according to Claim 12, **characterized in that** the at least one further fibre layer is hydrophilic.

14. Wound dressing according to one or more of the preceding claims, **characterized in that** the wound dressing and/or the needlefelt fabric has a shrinkage on contact with atmospheric moisture, measured as defined in the Measurement Methods section, after 24 hours of 0% to 10%, even more preferably of 0% to 5%, and/or the needlefelt fabric has an absorption capacity, measured as defined in the Measurement Methods section, for 0.9% aqueous sodium chloride solution of 1000% to 5000%, or of 1000% to 2500%, and/or the plastic component of the deformation $\varepsilon$(pl) of the wound dressing and/or of the needlefelt fabric, determined as defined in the Measurement Methods chapter, longitudinally and/or transversely, more preferably longitudinally and transversely, is less than 12%, preferably less than 10%, and more preferably less than 8%, and/or the rise height of 0.9% by weight NaCl solution in the wound dressing and/or in the needlefelt fabric, determined as defined in the Measurement Methods chapter, longitudinally and/or transversely, preferably longitudinally and transversely, is less than 30 mm, preferably less than 20 mm.

15. Method for producing a wound dressing, comprising the following steps:

    1) producing and/or providing a fibrous web containing 30% to 80% by weight, based on the total weight of the fibrous web, of superabsorbent fibres and 70% to 20% by weight, based on the total weight of the fibrous web, of self-crimping fibres;
    2) consolidating the fibrous web obtained in step 1) to give a needlefelt fabric by needling;
    3) initiating the self-crimping of the self-crimping fibres of the consolidated fibrous web obtained in step 2), preferably by thermal treatment, to obtain a wound dressing.

16. Use of a needlefelt fabric containing:

    - 30% to 80% by weight, based on the total weight of the needlefelt fabric, of superabsorbent fibres;
    - 70% to 20% by weight, based on the total weight of the needlefelt fabric, of self-crimped fibres,

    for producing a wound dressing.


**Revendications**

1. Pansement comprenant un non-tissé aiguilleté contenant :

    - 30% en poids à 80% en poids, par rapport au poids total du non-tissé aiguilleté, de fibres superabsorbantes ;
    - 70% en poids à 20% en poids, par rapport au poids total du non-tissé aiguilleté, de fibres auto-frisées.

2. Pansement selon la revendication 1, **caractérisé en ce que** le non-tissé aiguilleté est traité thermiquement.

3. Pansement selon la revendication 1 ou 2, **caractérisé en ce que** les fibres auto-frisées présentent des matériaux fibreux choisis dans le groupe des polyamides, par exemple le polyamide 6, le polyamide 6,6 ou le polyamide 11, des polyesters, par exemple le poly(téréphtalate d'éthylène) (PET) ou le poly(téréphtalate de butylène) (PBT), le poly(succinate de butylène) (PBS), et des polyoléfines, par exemple le polypropylène (PP) ou le polyéthylène (PE), ou parmi leurs mélanges ou copolymères.

**4.** Pansement selon l'une ou plusieurs des revendications précédentes, **caractérisé en ce que** les fibres auto-frisées sont fabriquées à partir de fibres auto-frisantes présentant au moins deux composants, un composant présentant un comportement de retrait différent sous traitement thermique de celui de l'autre composant et l'agencement des composants induisant une déformation tridimensionnelle de la fibre sous traitement thermique et/ou **en ce que** les fibres auto-frisées sont fabriquées à partir de fibres auto-frisantes, pour lesquelles il existe un comportement de retrait différent sous traitement thermique d'au moins un composant fibreux par rapport à celui d'au moins un autre composant fibreux.

**5.** Pansement selon l'une ou plusieurs des revendications précédentes, **caractérisé en ce que** les fibres auto-frisées et/ou les fibres auto-frisantes utilisées comme matériau de départ se trouvent sous forme de fibres à deux composants.

**6.** Pansement selon l'une ou plusieurs des revendications précédentes, **caractérisé en ce que** les fibres auto-frisées et les fibres superabsorbantes sont des fibres discontinues, la longueur des fibres discontinues des fibres auto-frisées et/ou des fibres superabsorbantes, indépendamment les unes des autres, étant de préférence située entre 32 et 80 mm et/ou le titre des fibres auto-frisées et/ou des fibres superabsorbantes, indépendamment les unes des autres, étant de préférence situé entre 1,7 et 20 dtex.

**7.** Pansement selon l'une ou plusieurs des revendications précédentes, **caractérisé en ce que** les fibres superabsorbantes contiennent des polymères choisis dans le groupe constitué par les polymères d'acides carboxyliques insaturés, les copolymères greffés d'acides carboxyliques insaturés, la cellulose modifiée, les polysaccharides gélifiants à l'exception de la cellulose modifiée, ainsi que leurs mélanges.

**8.** Pansement selon l'une ou plusieurs des revendications précédentes, **caractérisé en ce que** le non-tissé aiguilleté contient encore d'autres fibres, de préférence choisies parmi des fibres comprenant une ou plusieurs des matières premières fibreuses suivantes : polyoléfines, cellulose, cellulose régénérée, en particulier viscose, polyamides, polyacrylonitriles, élasthanes, poly(chlorures de vinyle), polylactides, polyglycolides, Polyesteramides, polycaprolactones, poly(téréphtalates d'hexaméthylène), polyhydroxybutyrates, polyhydroxyvalérates, fibres naturelles animales et/ou végétales et/ou polyesters.

**9.** Pansement selon la revendication 8, **caractérisé en ce que** les autres fibres sont des fibres hydrophiles, de préférence choisies parmi les fibres hydrophiles contenant une ou plusieurs matières premières fibreuses choisies parmi la cellulose, la cellulose régénérée, en particulier la viscose.

**10.** Pansement selon la revendication 8 ou 9, **caractérisé en ce que** les autres fibres sont présentes en une proportion de 5% en poids à 30% en poids, plus préférablement de 10% en poids à 20% en poids, par rapport au poids total du non-tissé aiguilleté.

**11.** Pansement selon l'une ou plusieurs des revendications précédentes, **caractérisé en ce que** le non-tissé aiguilleté présente une densité d'aiguilletage de 50 à 150 perforations/cm$^2$, de préférence de 70 à 120 perforations/cm$^2$, en particulier de 90 à 110 perforations/cm$^2$.

**12.** Pansement selon l'une ou plusieurs des revendications précédentes, **caractérisé en ce que** le pansement présente au moins une autre couche de fibres qui est agencée sur au moins un côté du non-tissé aiguilleté, ladite au moins une autre couche de fibres étant de préférence liée au non-tissé aiguilleté par aiguilletage.

**13.** Pansement selon la revendication 12, **caractérisé en ce que** ladite au moins une autre couche fibreuse est hydrophile.

**14.** Pansement selon l'une ou plusieurs des revendications précédentes, **caractérisé en ce que** le pansement et/ou le non-tissé aiguilleté présente(nt), au contact de l'humidité de l'air, un retrait mesuré comme défini dans la section des méthodes de mesure, après 24 heures, de 0% à 10%, plus préférablement de 0% à 5%, et/ou le non-tissé aiguilleté présente une capacité d'absorption, mesurée comme défini dans la section des méthodes de mesure, pour une solution aqueuse de chlorure de sodium à 0,9%, de 1000% à 5000% ou de 1000% à 2500% et/ou la proportion plastique de la déformation ε(pl) du pansement et/ou du non-tissé aiguilleté, déterminée comme défini dans le chapitre des méthodes de mesure, longitudinalement et/ou transversalement, de manière particulièrement préférée longitudinalement et transversalement, est inférieure à 12%, de préférence inférieure à 10%, de manière particulièrement préférée inférieure à 8%, et/ou la hauteur ascensionnelle d'une solution de NaCl à 0,9% en poids du pansement et/ou du non-tissé aiguilleté, déterminée comme défini dans le chapitre des méthodes de mesure, longitudinalement et/ou transversalement, de préférence longitudinalement et transversalement, est inférieure à 30 mm, de préférence inférieure à 20 mm.

**15.** Procédé de fabrication d'un pansement comprenant les étapes suivantes :

1) fabrication et/ou mise à disposition d'une nappe fibreuse comprenant 30% en poids à 80% en poids, par rapport au poids total de la nappe fibreuse, de fibres superabsorbantes et 70% en poids à 20% en poids, par rapport au poids total de la nappe fibreuse, de fibres auto-frisantes ;
2) consolidation de la nappe fibreuse obtenue à l'étape 1) afin d'obtenir un non-tissé aiguilleté par aiguilletage ;
3) déclenchement de l'auto-frisage des fibres auto-frisantes de la nappe fibreuse consolidée obtenue à l'étape 2), de préférence par traitement thermique, un pansement étant obtenu.

**16.** Utilisation d'un non-tissé aiguilleté contenant :

- 30% en poids à 80% en poids, par rapport au poids total du non-tissé aiguilleté, de fibres superabsorbantes ;
- 70% en poids à 20% en poids, par rapport au poids total du non-tissé aiguilleté, de fibres auto-frisées, pour la fabrication d'un pansement.

**Fig.1**

**Fig 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010035017 A1
- EP 3666295 A1
- EP 3285705 B1
- DE 102007049429 A1
- US 5454142 A
- US 4366206 A
- US 4788237 A
- EP 0680344 A
- WO 2011022740 A1
- EP 2196224 A1
- EP 1085912 A1